# EUROPEAN PATENT APPLICATION

(11) **EP 4 265 239 A1**
(43) Date of publication of application: **25.10.2023**
(21) Application number: 21907164.4
(22) Date of filing: 17.12.2021
(51) Int. Cl.: A61K 9/08, A61K 47/36, A61K 47/26, A61K 47/14, A61K 47/10, A61K 9/00, A61K 38/48

(54) **PHARMACEUTICAL COMPOSITION FOR LONG-TERM STORAGE OF LIQUID FORMULATION OF BOTULINUM TOXIN**

(30) Priority: 18.12.2020 KR 20200178961; 15.12.2021 KR 20210180076
(71) Applicant: ATGC Co., Ltd., Gangnam-gu Seoul 06372 (KR)
(72) Inventor: JANG, Sung Su, Seoul 05510 (KR); LIM, Il Ho, Seoul 05837 (KR); LEE, Hak Sup, Seongnam-si Gyeonggi-do 13533 (KR); AHN, Yong Shik, Seoul 02582 (KR); HWANG, Beom Ju, Incheon 21457 (KR)
(74) Representative: Nederlandsch Octrooibureau
(86) International application number: PCT/KR2021/019302
(87) International publication number: WO 2022/131860

(57) **Abstract**

The present invention relates to a composition for storing a liquid formulation of botulinum toxin for a long period of time at a low temperature and, more specifically, to a liquid-phase pharmaceutical composition and a method for maintaining biological activity of botulinum toxin by using same, wherein the composition comprises, as active ingredients, (i) botulinum toxin, (ii) hyaluronic acid or a pharmaceutically acceptable salt thereof, and (iii) one or more additives selected from the group consisting of saccharides, a non-ionic surfactant, and a stabilizer. The composition according to the present invention has a remarkable effect of physically and chemically stabilizing botulinum toxin and preventing the tertiary structure of botulinum toxin from denaturing, thus making it possible to expand the formulation of botulinum toxin from conventionally limited dry agents to liquid-phase agents.

## Description

### [Technical Field]

The present invention relates to a composition for long-term storage of a liquid botulinum toxin formulation at low temperature, specifically, a pharmaceutical liquid composition comprising as active ingredients (i) botulinum toxin, (ii) hyaluronic acid or a pharmaceutically acceptable salt thereof, and (iii) one or more additives selected from the group consisting of sugars, non-ionic surfactants, and stabilizers, and a method for maintaining the biological activity of botulinum toxin using the same.

This application claims priority based on Korean Patent Application No. 10-2020-0178961 filed on December 18, 2020, and Korean Patent Application No. 10-2021-0180076 filed on December 15, 2021, and all contents disclosed in the specification and drawings of the respective applications are incorporated herein by reference.

### [Background Art]

Botulinum toxin is a neurotoxin produced by the Gram-positive anaerobic bacterium *Clostridium botulinum.* Botulinum toxin is classified into eight neurotoxins, seven of which (types A, B, C, D, E, F, and G) can cause paralysis of the nerves. Among them, the most lethal botulinum toxin known as a natural biological agent is type A, with the size of the toxin protein being about 150 kDa. In addition to the toxin protein, nontoxic complexing proteins are combined to form a complex, and the size of the complex can be formed up to 900 kDa depending on the type of neurotoxin.

Botulinum toxin has an effect of causing temporary relaxation paralysis of muscles, and its mechanism of action is known to cause local muscle paralysis by inhibiting the secretion of acetylcholine at the neuromuscular junction of the motor nerve endings (entering nerve cells by binding to the cholinergic endings of peripheral nerves). Botulinum toxin has an effect of suppressing pain caused by chronic myofascial pain, low back pain, muscle stiffness, and tension headaches by inducing localized muscle paralysis, which is believed to be due to the inhibition of acetylcholine release from the axon terminal of the neuromuscular junction, as known in the above mechanism of action, thereby blocking nerve signal transmission and resulting in pain suppression.

Botulinum toxin has been widely used for the purpose of improving strabismus and forehead wrinkles since it was approved by the US Food and Drug Administration (FDA) in 1989. Therapeutically, it is used for strabismus, facial tremors, eyelid spasms, muscle stiffness, and for cosmetic purposes, it is used for wrinkle removal and square jaw procedures.

The duration of botulinum toxin injected into muscle and skin tissues is within 3 to 6 months, and the injection effect begins within 3 days and the maximum effect appears between 1 and 2 weeks. As the nerve signal transmission is blocked by the inhibition of acetylcholine secretion at the neuromuscular junction by botulinum toxin, and then the effect of nerve paralysis by botulinum toxin is reduced as new nerve branches are generated, regular administration of botulinum toxin is essential.

Meanwhile, hyaluronic acid is an anionic non-sulfated glycosaminoglycan (GAGs) widely distributed in connective, epithelial, and neural tissues, and is one of the main components of the extracellular matrix. It mainly contributes to cell proliferation and migration and is used as one of the biocompatible biomaterials for the purpose of treating diseases such as osteoarthritis as a main component of pharmaceuticals or as an additive for providing viscosity in liquid formulations.

Protein pharmaceuticals such as botulinum toxin have a very narrow range of formulation choices due to the influence of tertiary structure denaturation on the pharmacological activity of the drug. In particular, among various factors affecting the tertiary structure denaturation of botulinum toxin, the moisture content in the finished product has a significant impact on the stability of efficacy. This is one of the reasons why existing botulinum toxin formulations are limited to vacuum-dried injectables or freeze-dried injectables, where moisture in the finished product is sufficiently removed.

Moreover, vacuum-dried injectables or freeze-dried injectables require users to dilute them for use, leading to potential safety issues due to variations in the administration of the main component depending on the user's dilution method or management state, and also have the disadvantage of reduced user convenience.

Therefore, it is crucial to develop a liquid formulation that can maintain the pharmacological activity of the enclosed botulinum toxin while enhancing user convenience. Many prior studies have been conducted to develop liquid stabilizing formulations for botulinum toxin in response to such demand, but most of the research results have failed to maintain the initial biological activity of botulinum toxin. Consequently, low-temperature storage to ensure the pharmacological stability of botulinum toxin contained in liquid formulations is being spotlighted as the most realistic alternative, and there remains a technical challenge to overcome in securing the low-temperature storage stability with a novel additive that can maintain the pharmacological activity of botulinum toxin in the liquid formulation.

### [Disclosure]

### [Technical Problem]

The purpose of the present invention is to provide a pharmacological liquid composition comprising as active ingredients: (i) botulinum toxin; (ii) hyaluronic acid or a pharmaceutically acceptable salt thereof; and (iii) one or more additives selected from a group consisting of sugars, non-ionic surfactants, and stabilizers.

Another purpose of the present invention is to provide a method for maintaining the biological activity of botulinum toxin using the composition according to the present invention.

However, the technical problems to be achieved by the present invention are not limited to the problems mentioned above, and other unmentioned problems can be clearly understood by those skilled in the art to which the present invention pertains from the description below.

### [Technical Solution]

To achieve the objectives of the present invention, the present invention provides a pharmacological liquid composition comprising as active ingredients: (i) botulinum toxin; (ii) hyaluronic acid or a pharmaceutically acceptable salt thereof; and (iii) one or more additives selected from a group consisting of sugars, non-ionic surfactants, and stabilizers.

In addition, the present invention provides a method for maintaining the biological activity of botulinum toxin using the composition according to the present invention.

The inventors have completed the present invention by developing a pharmacological liquid composition containing hyaluronic acid and additives, which enables stable storage without a decrease in biological activity even when the liquid formulation of botulinum toxin is stored at low temperatures for an extended period.

In one embodiment of the present invention, the composition may contain botulinum toxin at a concentration of 5 to 40 units/mL based on the total composition, but is not limited thereto.

In another embodiment of the present invention, the botulinum toxin may be in a form without complexed proteins or in a complex form containing complexed proteins, but is not limited thereto.

In yet another embodiment of the present invention, the molecular weight of the hyaluronic acid may be between 30,000 and 5,000,000 Daltons (Da), but is not limited thereto.

In yet another embodiment of the present invention, the composition may contain hyaluronic acid at a concentration of 5 to 250 mg/mL based on the total composition, but is not limited thereto.

In yet another embodiment of the present invention, the sugar may be lactose monohydrate or fructose, but is not limited thereto.

In yet another embodiment of the present invention, the composition may contain the sugar at a concentration of 0.01 to 5% (w/v) based on the total composition, but is not limited thereto.

In yet another embodiment of the present invention, the non-ionic surfactant may be polyoxyethylene hydrogenated castor oil, but is not limited thereto.

In yet another embodiment of the present invention, the polyoxyethylene hydrogenated castor oil may have a molar ratio of ethylene oxide between 10 and 60, but is not limited thereto.

In yet another embodiment of the present invention, the composition may contain the non-ionic surfactant at a concentration of 0.01 to 1% (w/v) based on the total composition, but is not limited thereto.

In yet another embodiment of the present invention, the stabilizer may be glycerin, but is not limited thereto.

In yet another embodiment of the present invention, the composition may contain the stabilizer at a concentration of 0.1 to 10% (w/v) based on the total composition.

In yet another embodiment of the present invention, the hyaluronic acid may have a molecular weight between 500,000 and 2,000,000 Da; the sugar may not be present or may be present at a concentration of 0.1 to 5% (w/v) based on the total composition; the non-ionic surfactant may be present at a concentration of 0.01 to 1% (w/v) based on the total composition; and the stabilizer may not be present or may be present at a concentration of 0.1 to 10% (w/v) based on the total composition, but is not limited thereto.

In yet another embodiment of the present invention, the hyaluronic acid may have a molecular weight between 500,000 and 2,000,000 Da; the sugar may be present at a concentration of 0.1 to 5% (w/v) based on the total composition; the non-ionic surfactant may not be present or may be present at a concentration of 0.01 to 1% (w/v) based on the total composition; and the stabilizer may not be present or may be present at a concentration of 0.1 to 10% (w/v) based on the total composition, but is not limited thereto.

In yet another embodiment of the present invention, the hyaluronic acid may have a molecular weight between 2,500,000 and 5,000,000 Da;
the sugar may not be present or may be present at a concentration of 0.1 to 5% (w/v) based on the total composition;
the non-ionic surfactant may be present at a concentration of 0.01 to 1% (w/v) based on the total composition; and
the stabilizer may not be present or may be present at a concentration of 0.1 to 1% (w/v) based on the total composition, but is not limited thereto.

In yet another embodiment of the present invention, the composition may further include 25 to 100 mM of sodium phosphate as a buffering agent.

In yet another embodiment of the present invention, the composition may be a ready-to-use injectable formulation, but is not limited thereto.

Additionally, the present invention provides a method for manufacturing a long-term storage pharmaceutical liquid composition of botulinum toxin, comprising a step of mixing botulinum toxin; hyaluronic acid or a pharmaceutically acceptable salt thereof; and one or more additives selected from the group consisting of sugars, non-ionic surfactants, and stabilizers.

Furthermore, the present invention provides a stabilization use of botulinum toxin in a liquid composition comprising hyaluronic acid or a pharmaceutically acceptable salt thereof; and one or more additives selected from the group consisting of sugars, non-ionic surfactants, and stabilizers. The stabilization use includes maintaining the biological activity of the botulinum toxin.

Moreover, the present invention provides a use of a composition comprising hyaluronic acid or a pharmaceutically acceptable salt thereof; and one or more additives selected from the group consisting of sugars, non-ionic surfactants, and stabilizers for manufacturing a liquid stabilizer for botulinum toxin.

Additionally, the present invention provides a pharmaceutical composition for the prevention or treatment of neuromuscular-related diseases, comprising the liquid composition according to the present invention as an active ingredient.

Moreover, the present invention provides a cosmetic composition for the prevention or improvement of neuromuscular-related diseases, comprising the liquid composition according to the present invention as an active ingredient.

In one embodiment of the present invention, the neuromuscular-related diseases may be selected from a group consisting of headache, migraine, tension headache, sinus headache, cervicogenic headache, hyperhidrosis, axillary hyperhidrosis, palmar hyperhidrosis, plantar hyperhidrosis, Frey syndrome, hyperkinetic skin lines, facial wrinkles, forehead wrinkles, crow's feet, perioral wrinkles, nasolabial wrinkles, skin disorders, acne, rhinitis, sinusitis, achalasia, strabismus, chronic anal fissure, blepharospasm, musculoskeletal pain, foot pain, plantar fasciitis, fibromyalgia, pancreatitis, tachycardia, prostatic hypertrophy, prostatitis, urinary retention, urinary incontinence, overactive bladder, hemifacial spasm, tremor, muscle spasms, gastrointestinal disorders, diabetes, gout, sialorrhea, detrusor-sphincter dyssynergia, post-stroke spasticity, wound healing, cerebral palsy in children, smooth muscle spasms, restenosis, focal dystonia, epilepsy, cervical dystonia, thyroid disorders, hypercalcemia, obsessive-compulsive disorder, osteoarthritis, temporomandibular joint disorder, Raynaud's syndrome, striae distensae, peritoneal adhesions, vasospasm, rhinorrhea, muscle contractures, laryngeal dystonia, writer's cramp, and carpal tunnel syndrome, but is not limited thereto.

Additionally, the present invention provides a method for preventing or treating neuromuscular-related diseases, comprising the step of administering the liquid composition according to the present invention to a subject in need thereof.

Furthermore, the present invention provides a use of the liquid composition according to the present invention for the prevention or treatment of neuromuscular-related diseases.

Moreover, the present invention provides a use of the liquid composition for manufacturing a therapeutic agent for neuromuscular-related diseases.

### [Advantageous Effects]

The present invention provides a liquid composition containing an additive for maintaining the pharmacological activity of botulinum toxin and hyaluronic acid, which are active ingredients, thereby significantly increasing the stability of botulinum toxin during long-term storage. By combining additives such as pH adjusters, sugars, surfactants, and stabilizers at specific concentrations and adding them to the botulinum toxin liquid formulation, the composition exhibits excellent effects in preventing physical and chemical instability and denaturation of the 3D structure of botulinum toxin. Therefore, by using the composition according to the present invention, the range of applicable formulations of botulinum toxin, which has been limited to dry formulations, can be expanded to liquid formulations, enabling the development of various types of products.

Furthermore, the stability enhancement of botulinum toxin in the liquid composition utilizing the present invention can be used to ensure stability during the formulation process of botulinum toxin, applying it to controlled-release injectable drug formulations in sustained-release or immediate-release forms, or utilizing it for the development of transdermal pharmaceuticals using microstructures such as microneedle patches. Additionally, the excellent biocompatibility of the active ingredient, hyaluronic acid, provides an additional anti-inflammatory effect and imparts viscosity to the finished product, allowing for an expectation of localized therapeutic effects of botulinum toxin.

### [Description of the Drawings]

FIG. 1 is a graph showing the low-temperature stability of the botulinum toxin liquid composition according to the type and concentration of sugar used as an additive (added hyaluronic acid molecular weight = 1 MDa).
FIG. 2 is a graph showing the low-temperature stability of the botulinum toxin liquid composition according to the concentration of non-ionic surfactant used as an additive (added hyaluronic acid molecular weight = 1 MDa).
FIG. 3 is a graph showing the low-temperature stability of the botulinum toxin liquid composition according to the concentration of stabilizer used as an additive (added hyaluronic acid molecular weight = 1 MDa).
FIG. 4 is a graph comparing the stabilization efficacy of the botulinum toxin liquid composition after adding various additives at different concentrations (added hyaluronic acid molecular weight = 1 MDa).
FIG. 5 is a graph comparing the stabilization efficacy of the botulinum toxin liquid composition after adding various additives at different concentrations (added hyaluronic acid molecular weight = 3 MDa).

### [Best Modes of the Invention]

The inventors have specifically confirmed that the pharmacological activity of botulinum toxin in a liquid formulation containing botulinum toxin, hyaluronic acid, and additives can be maintained at low temperatures for an extended period using the composition according to the present invention and completed the present invention.

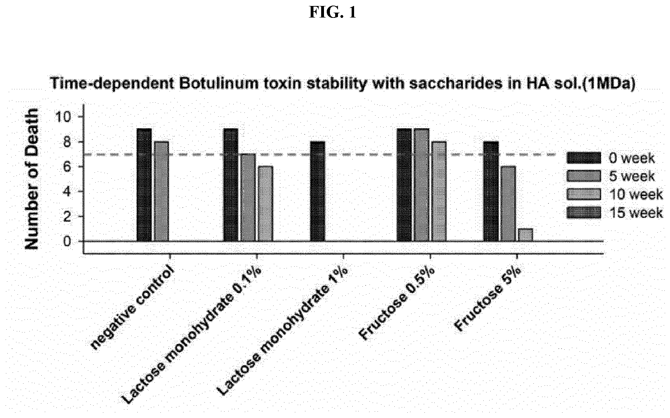

In one embodiment of the present invention, screening experiments were performed for various types of sugars, and lactose monohydrate and fructose were selected as additives. As a result of evaluating the stability of the botulinum toxin liquid composition by adding them at various concentrations, it was confirmed that the low-temperature stability of the test group containing 0.1% (w/v) lactose monohydrate and the test group containing 0.5% (w/v) fructose was significantly improved (Example 1, Table 1, and FIG. 1).

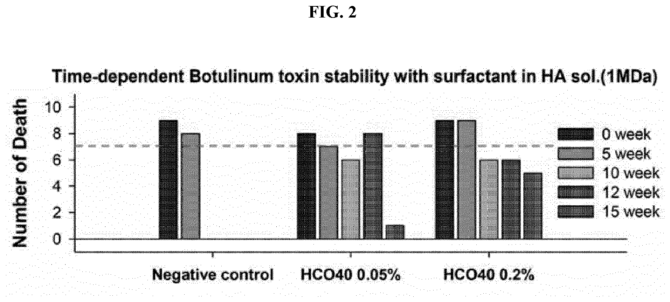

In another embodiment of the present invention, screening experiments were performed for various types of non-ionic surfactants, and polyoxyethylene hardened castor oil was selected as an additive. As a result of evaluating the stability of the botulinum toxin liquid composition by adding it at various concentrations, it was confirmed that the low-temperature stability could be maintained up to 15 weeks in the test group containing 0.2% (w/v) polyoxyethylene hardened castor oil (Example 2, Table 2, and FIG. 2).

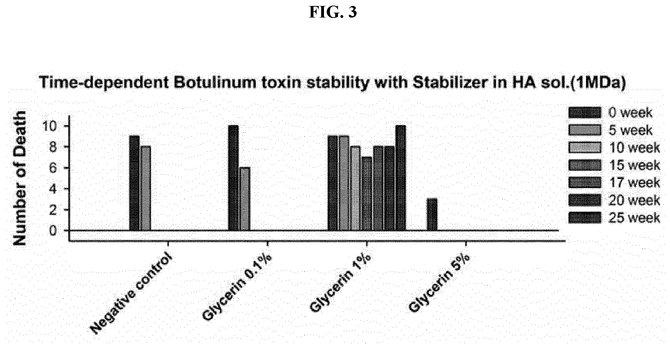

In still another embodiment of the present invention, screening experiments were performed for various types of stabilizers, and glycerin was selected as an additive. As a result of evaluating the stability of the botulinum toxin liquid composition by adding it at various concentrations, it was confirmed that the low-temperature stability could be maintained up to 25 weeks in the test group containing 1% (w/v) glycerin (Example 3, Table 3, and FIG. 3).

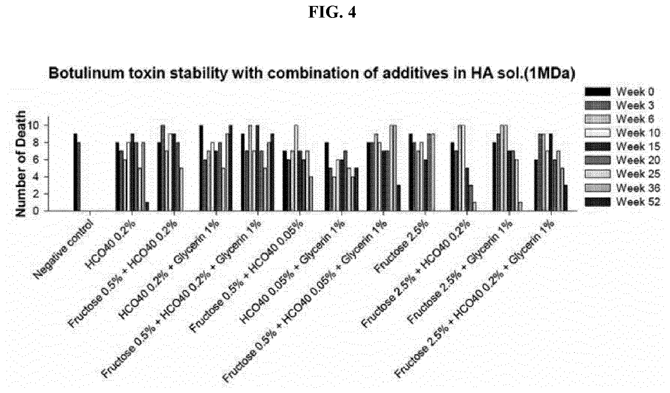

In yet another embodiment of the present invention, based on the above embodiments, the stabilization efficacy of the composition mixed with hyaluronic acid of 1 MDa and various combinations of additives was confirmed, and it was found that the stabilization efficacy of botulinum toxin in the compositions satisfying the following additives was particularly excellent: additives containing HCO-40 0.2% (w/v); additives containing fructose 0.5% (w/v), HCO-40 0.2% (w/v); additives containing HCO-40 0.2% (w/v), glycerin 1% (w/v); additives containing fructose 0.5% (w/v), HCO-40 0.2% (w/v), glycerin 1% (w/v); and additives containing fructose 0.5% (w/v), HCO-40 0.05% (w/v), glycerin 1% (w/v) (Example 4, Table 4, and FIG. 4).

In particular, in the above embodiments, compositions containing additives with HCO-40 0.2% (w/v), glycerin 1% (w/v); and additives containing fructose 0.5% (w/v), HCO-40 0.2% (w/v), glycerin 1% (w/v) were confirmed to achieve long-term botulinum toxin stability maintenance effects for more than 12 months.

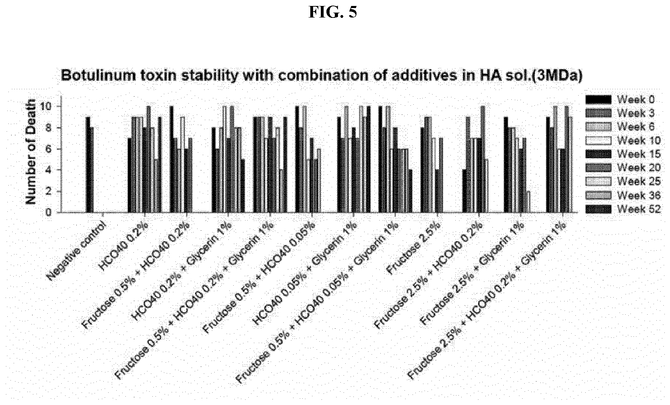

In yet another embodiment of the present invention, based on the above embodiments, the stabilization efficacy of the composition mixed with hyaluronic acid of 3 MDa and various combinations of additives was confirmed. As a result, it was found that the stabilization efficacy of botulinum toxin in the compositions containing HCO-40 0.2% (w/v) additives; additives containing fructose 0.5% (w/v), HCO-40 0.2% (w/v), glycerin 1% (w/v); and additives containing HCO-40 0.05% (w/v), glycerin 1% (w/v) was particularly excellent, and long-term botulinum toxin stability maintenance effects for more than 12 months could be achieved (Example 5, Table 5, and FIG. 5).

Therefore, the present invention can provide a pharmaceutical liquid composition comprising as active ingredients: (i) botulinum toxin; (ii) hyaluronic acid or its pharmaceutically acceptable salt; and (iii) one or more additives selected from the group consisting of sugars, non-ionic surfactants, and stabilizers.

In another aspect of the present invention, there is provided a method for maintaining the biological activity of botulinum toxin using a pharmaceutical liquid composition comprising as active ingredients: (i) botulinum toxin; (ii) hyaluronic acid or its pharmaceutically acceptable salt; and (iii) one or more additives selected from the group consisting of sugars, non-ionic surfactants, and stabilizers.

The pharmaceutical liquid composition according to the present invention can desirably maintain the biological activity of botulinum toxin for 12 months or more, 10 months or more, 8 months or more, 6 months or more, or 5 months or more, but is not limited thereto.

In the present specification, the term "botulinum toxin" refers to a toxin produced by bacteria or by recombinant techniques, encompassing any disclosed type of botulinum toxin, manipulated variants or fusion proteins thereof, or any type of botulinum toxin that may be discovered subsequently. Botulinum toxin is classified into eight serotypes, and the serotypes A, B, C, D, E, F, and G can induce paralysis. The botulinum toxin according to the present invention may be botulinum toxin type A, but is not limited thereto.

The botulinum toxin includes all serotypes and derivatives or fusion proteins thereof, produced by bacteria or recombinant techniques. In the present invention, "botulinum toxin derivatives" may refer to derivatives comprising one or more chemical or functional modifications on a part or some chains of natural botulinum toxin or recombinant prototype botulinum toxin having botulinum toxin activity. For example, botulinum toxin derivatives may be modified toxins having one or more deleted, altered, or substituted amino acids.

In the present invention, the composition may include botulinum toxin at a concentration of 5 to 40 units/mL based on the total composition.

One unit (U) of the botulinum toxin can be defined as the LD50, which is the lethal dose at which 50% of the female Swiss Webster mice weighing 17 to 22 g die through intraperitoneal injection. The LD50 of botulinum toxin serotype A in mice is approximately 50 picograms.

In the present invention, the botulinum toxin may be in a form without a complexing protein or in a complex form containing a complexing protein. The botulinum toxin can be divided into proteins with and without a complex, with the molecular weight of the pure toxin protein being approximately 150 kDa, and proteins of various sizes, such as 300 kDa, 500 kDa, and 900 kDa, are generated depending on the presence or absence of the complex.

In the present invention, the molecular weight of the hyaluronic acid may be 30,000 to 5,000,000 Da, but is not limited thereto. For example, the composition according to the present invention may comprise hyaluronic acid as an active ingredient with a molecular weight of 30,000 to 5,000,000 Da, 30,000 to 4,500,000 Da, 30,000 to 4,000,000 Da, 30,000 to 3,500,000 Da, 30,000 to 3,000,000 Da, 500,000 to 5,000,000 Da, 500,000 to 4,500,000 Da, 500,000 to 4,000,000 Da, 500,000 to 3,500,000 Da, 500,000 to 3,000,000 Da, 1,000,000 to 5,000,000 Da, 1,000,000 to 4,500,000 Da, 1,000,000 to 4,000,000 Da, 1,000,000 to 3,500,000 Da, 1,000,000 to 3,000,000 Da, 500,000 to 2,000,000 Da, 500,000 to 1,800,000 Da, 500,000 to 1,700,000 Da, 500,000 to 1,500,000 Da, 700,000 to 1,300,000 Da, 800,000 to 1,200,000 Da, 900,000 to 1,100,000 Da, 1,500,000 to 5,000,000 Da, 2,000,000 to 5,000,000 Da, 2,500,000 to 5,000,000 Da, 2,500,000 to 4,500,000 Da, 2,500,000 to 4,000,000 Da, 2,500,000 to 3,500,000 Da, 2,700,000 to 3,300,000 Da, 2,800,000 to 3,200,000 Da, or 2,900,000 to 3,100,000 Da.

In the present invention, the composition may comprise hyaluronic acid at a concentration of 5 to 250 mg/mL based on the total composition, but is not limited thereto. For example, the composition may comprise hyaluronic acid at concentrations of 5 to 250 mg/mL, 10 to 250 mg/mL, 15 to 250 mg/mL, 20 to 250 mg/mL, 30 to 250 mg/mL, 40 to 250 mg/mL, 50 to 250 mg/mL, 70 to 250 mg/mL, 90 to 250 mg/mL, 100 to 250 mg/mL, 150 to 250 mg/mL, 200 to 250 mg/mL, 5 to 200 mg/mL, 10 to 200 mg/mL, 15 to 200 mg/mL, 20 to 200 mg/mL, 30 to 200 mg/mL, 40 to 200 mg/mL, 50 to 200 mg/mL, 70 to 200 mg/mL, 90 to 200 mg/mL, 100 to 200 mg/mL, 150 to 200 mg/mL, 5 to 150 mg/mL, 10 to 150 mg/mL, 15 to 150 mg/mL, 20 to 150 mg/mL, 30 to 150 mg/mL, 40 to 150 mg/mL, 50 to 150 mg/mL, 70 to 150 mg/mL, 90 to 150 mg/mL, 100 to 150 mg/mL, 5 to 100 mg/mL, 10 to 100 mg/mL, 15 to 100 mg/mL, 20 to 100 mg/mL, 30 to 100 mg/mL, 40 to 100 mg/mL, 50 to 100 mg/mL, 70 to 100 mg/mL, 90 to 100 mg/mL, 5 to 90 mg/mL, 10 to 90 mg/mL, 15 to 90 mg/mL, 20 to 90 mg/mL, 30 to 90 mg/mL, 40 to 90 mg/mL, 50 to 90 mg/mL, 70 to 90 mg/mL, 5 to 70 mg/mL, 10 to 70 mg/mL, 15 to 70 mg/mL, 20 to 70 mg/mL, 30 to 70 mg/mL, 40 to 70 mg/mL, 50 to 70 mg/mL, 5 to 50 mg/mL, 10 to 50 mg/mL, 15 to 50 mg/mL, 20 to 50 mg/mL, 30 to 50 mg/mL, 40 to 50 mg/mL, 5 to 40 mg/mL, 10 to 40 mg/mL, 15 to 40 mg/mL, 20 to 40 mg/mL, 30 to 40 mg/mL, 5 to 30 mg/mL, 10 to 30 mg/mL, 15 to 30 mg/mL, 20 to 30 mg/mL, 5 to 20 mg/mL, 10 to 20 mg/mL, 15 to 20 mg/mL, 5 to 15 mg/mL, 10 to 15 mg/mL, or 5 to 10 mg/mL. The hyaluronic acid may be dissolved in a sodium chloride solution, but is not limited thereto.

In the present specification, the term "hyaluronic acid" refers to a complex polysaccharide composed of amino acids and uronic acids, having various molecular weights, and hyaluronic acid having the same molecular weight as that present in the human body is not only biologically safe but also effective in reducing inflammatory levels and restoring the rheology of synovial fluid when injected subcutaneously.

The term "pharmaceutically acceptable salts" as used herein generally refers to salts that are recognized for use in animals, particularly humans. And it includes inorganic salts formed by using bases to form salts, such as lithium, sodium, potassium, calcium, magnesium, or aluminum salts; organic salts formed by using bases, such as ethylamine, diethylamine, ethylene diamine, ethanolamine, diethanolamine, arginine, lysine, histidine, or piperazine; organic salts formed by using acids to form salts, such as acetate, citrate, lactate, malonate, maleate, tartrate, fumarate, benzoate, aspartate, glutamate, succinate, oleate, trifluoroacetate, oxalate, pamoate, or gluconate; and inorganic salts such as chloride, sulfate, borate, or carbonate, but is not limited thereto. The characteristics of the salt are not an important factor, provided that the salt is pharmaceutically acceptable. The pharmaceutically acceptable salts of hyaluronic acid in the composition of the present invention can be obtained by conventional methods well known in the relevant technical field.

In the present invention, the sugar may be a monosaccharide (2 to 7 saccharide units) or a polysaccharide. There is no limitation on the type of sugar, but it can be selected from glucose, fructose, galactose, sucrose, lactose, cellulose, and starch, among others. Preferably, in the present invention, the sugar may be lactose monohydrate or fructose.

In the present invention, the composition may contain the sugar at a concentration of 0.01 to 5% (w/v) based on the total composition, but is not limited thereto. For example, the sugar may be contained in the composition at a concentration of 0.01 to 5% (w/v), 0.05 to 5% (w/v), 0.1 to 5% (w/v), 0.5 to 5% (w/v), 1 to 5% (w/v), 1.5 to 5% (w/v), 2 to 5% (w/v), 2.5 to 5% (w/v), 0.01 to 4.5% (w/v), 0.05 to 4.5% (w/v), 0.1 to 4.5% (w/v), 0.5 to 4.5% (w/v), 1 to 4.5% (w/v), 1.5 to 4.5% (w/v), 2 to 4.5% (w/v), 2.5 to 4.5% (w/v), 0.01 to 4% (w/v), 0.05 to 4% (w/v), 0.1 to 4% (w/v), 0.5 to 4% (w/v), 1 to 4% (w/v), 1.5 to 4% (w/v), 2 to 4% (w/v), 2.5 to 4% (w/v), 0.01 to 3.5% (w/v), 0.05 to 3.5% (w/v), 0.1 to 3.5% (w/v), 0.5 to 3.5% (w/v), 1 to 3.5% (w/v), 1.5 to 3.5% (w/v), 2 to 3.5% (w/v), 2.5 to 3.5% (w/v), 0.01 to 3% (w/v), 0.05 to 3% (w/v), 0.1 to 3% (w/v), 0.2 to 2.8% (w/v), 0.4 to 2.6% (w/v), 0.5 to 3% (w/v), 1 to 3% (w/v), 1.5 to 3% (w/v), 2 to 3% (w/v), 2.5 to 3% (w/v), 0.01 to 2.5% (w/v), 0.05 to 2.5% (w/v), 0.1 to 2.5% (w/v), 0.5 to 2.5% (w/v), 1 to 2.5% (w/v), 1.5 to 2.5% (w/v), or 2 to 2.5% (w/v). The sugar may be dissolved in a sodium phosphate buffer, but is not limited thereto.

In the present invention, the term "non-ionic surfactant" refers to a surfactant that does not ionize in water and has excellent effects in dispersion, penetration, and emulsification. Due to its weak hydrophilicity, it causes less skin irritation and has excellent emulsification properties, making it useful not only as an excipient for pharmaceuticals but also as a cosmetic ingredient. For instance, non-ionic surfactant components include polyethylene glycol, cetyl alcohol, stearyl alcohol, fatty alcohol, decyl glucoside, lauryl glucoside, octyl glucoside, polysorbate, cocamide, cocamide MEA, and cocamide DEA.

There is no limitation on the specific types of non-ionic surfactants according to the present invention, but they may be selected from polysorbate (e.g., polysorbate 20, 40, 60, 80), polyoxyethylene hydrogenated castor oil, octyldodeceth-16, and ceteareth-6 olivate. Preferably, in the present invention, the non-ionic surfactant may be polyoxyethylene hydrogenated castor oil.

In the present invention, the polyoxyethylene hydrogenated castor oil may have a molar ratio of ethylene oxide of 10 to 60, but is not limited thereto. Specifically, castor oil to which ethylene oxide has been added by reacting castor oil with ethylene oxide can be used, wherein the ethylene oxide may be 10 to 60 mol based on 1 mol of castor oil. In one specific example, polyoxyethylene (40) hydrogenated castor oil was obtained by addition polymerization through adding 40 mol of ethylene oxide to hydrogenated (i.e., hydrogen-added) castor oil. The term "polyoxyethylene (40) hydrogenated castor oil" was used interchangeably with the term "HCO-40". However, this is just one specific example, and polyoxyethylene hydrogenated castor oil according to the present invention can be selected without limitation from HCO-10, HCO-20, HCO-30, HCO-40, HCO-50, and HCO-60.

In the present invention, the composition may contain the non-ionic surfactant at a concentration of 0.01 to 1% (w/v) based on the total composition, but is not limited thereto. For example, the non-ionic surfactant may be contained in the composition at concentrations of 0.01 to 1% (w/v), 0.01 to 0.9% (w/v), 0.01 to 0.8% (w/v), 0.01 to 0.7% (w/v), 0.01 to 0.6% (w/v), 0.01 to 0.5% (w/v), 0.01 to 0.4% (w/v), 0.01 to 0.3% (w/v), 0.03 to 1% (w/v), 0.03 to 0.9% (w/v), 0.03 to 0.8% (w/v), 0.03 to 0.7% (w/v), 0.03 to 0.6% (w/v), 0.03 to 0.5% (w/v), 0.03 to 0.4% (w/v), 0.03 to 0.3% (w/v), 0.05 to 1% (w/v), 0.05 to 0.8% (w/v), 0.1 to 0.8% (w/v), 0.15 to 0.8% (w/v), 0.2 to 0.8% (w/v), 0.05 to 0.7% (w/v), 0.1 to 0.7% (w/v), 0.15 to 0.7% (w/v), 0.2 to 0.7% (w/v), 0.05 to 0.6% (w/v), 0.1 to 0.6% (w/v), 0.15 to 0.6% (w/v), 0.2 to 0.6% (w/v), 0.05 to 0.5% (w/v), 0.1 to 0.5% (w/v), 0.15 to 0.5% (w/v), 0.2 to 0.5% (w/v), 0.05 to 0.4% (w/v), 0.1 to 0.4% (w/v), 0.15 to 0.4% (w/v), 0.2 to 0.4% (w/v), 0.05 to 0.3% (w/v), 0.1 to 0.3% (w/v), 0.15 to 0.3% (w/v), 0.2 to 0.3% (w/v), 0.05 to 0.25% (w/v), 0.1 to 0.25% (w/v), 0.15 to 0.25% (w/v), or 0.2 to 0.25% (w/v). The non-ionic surfactant may be dissolved in a sodium phosphate buffer for use, but is not limited thereto.

In the present invention, the term "stabilizer" refers to any additive for increasing the stability of the active ingredient, preventing the active ingredient from undergoing arbitrary changes, such as oxidation, crystallization, or denaturation into a flexible material, and the like. The evaluation of the stabilizing efficacy of the stabilizer can be performed without limiting the temperature, but the present invention is particularly understood to maintain the stabilizing effect of botulinum toxin at low temperatures over a long term rather than at high temperatures.

In the present invention, the stabilizer may be glycerin, but is not limited thereto.

In the present invention, the composition may contain the stabilizer at a concentration of 0.1 to 10% (w/v) based on the total composition, but is not limited thereto. For example, the stabilizer may be contained in the composition at concentrations of 0.1 to 10% (w/v), 0.5 to 10% (w/v), 1 to 10% (w/v), 1.5 to 10% (w/v), 2 to 10% (w/v), 3 to 10% (w/v), 4 to 10% (w/v), 5 to 10% (w/v), 7 to 10% (w/v), 9 to 10% (w/v), 0.1 to 9% (w/v), 0.5 to 9% (w/v), 1 to 9% (w/v), 1.5 to 9% (w/v), 2 to 9% (w/v), 3 to 9% (w/v), 4 to 9% (w/v), 5 to 9% (w/v), 7 to 9% (w/v), 0.1 to 7% (w/v), 0.5 to 7% (w/v), 1 to 7% (w/v), 1.5 to 7% (w/v), 2 to 7% (w/v), 3 to 7% (w/v), 4 to 7% (w/v), 5 to 7% (w/v), 0.1 to 5% (w/v), 0.5 to 5% (w/v), 1 to 5% (w/v), 1.5 to 5% (w/v), 2 to 5% (w/v), 3 to 5% (w/v), 4 to 5% (w/v), 0.1 to 4% (w/v), 0.5 to 4% (w/v), 1 to 4% (w/v), 1.5 to 4% (w/v), 2 to 4% (w/v), 3 to 4% (w/v), 0.1 to 3% (w/v), 0.5 to 3% (w/v), 1 to 3% (w/v), 1.5 to 3% (w/v), 2 to 3% (w/v), 0.1 to 2% (w/v), 0.5 to 2% (w/v), 1 to 2% (w/v), 1.5 to 2% (w/v), 0.1 to 1.5% (w/v), 0.5 to 1.5% (w/v), 1 to 1.5% (w/v), 0.1 to 1% (w/v), 0.5 to 1% (w/v), 0.1 to 0.5% (w/v), 0.2 to 2% (w/v), 0.4 to 1.8% (w/v), 0.6 to 1.6% (w/v), 0.8 to 1.4% (w/v), or 0.8 to 1.2% (w/v). The stabilizer may be dissolved in a sodium phosphate buffer for use, but is not limited thereto.

Preferably, in the composition according to the present invention, when the composition does not contain a sugar, the concentration ratio of the non-ionic surfactant and the stabilizer included in the composition may be 0.01 to 1:1 (w/v), 0.01 to 0.9:1 (w/v), 0.01 to 0.8:1 (w/v), 0.01 to 0.7:1 (w/v), 0.01 to 0.6:1 (w/v), 0.01 to 0.5:1 (w/v), 0.01 to 0.4:1 (w/v), or 0.01 to 0.3:1 (w/v), but is not limited thereto.

Preferably, in the composition according to the present invention, when the composition does not contain a non-ionic surfactant, the concentration ratio of the sugar and the stabilizer included in the composition may be 1 to 10:1 (w/v), 1 to 9:1 (w/v), 1 to 8:1 (w/v), 1 to 7:1 (w/v), 1 to 6:1 (w/v), 1 to 5:1 (w/v), 1 to 4:1 (w/v), 1 to 3:1 (w/v), 2 to 5:1 (w/v), 2 to 4:1 (w/v), or 2 to 3:1 (w/v), but is not limited thereto.

Preferably, in the composition according to the present invention, when the composition does not contain a stabilizer, the concentration ratio of the sugar and the non-ionic surfactant included in the composition may be 1 to 20:1 (w/v), 1 to 18:1 (w/v), 1 to 16:1 (w/v), 1 to 14:1 (w/v), 1 to 13:1 (w/v), 1 to 10:1 (w/v), 1 to 9:1 (w/v), 1 to 8:1 (w/v), 1 to 7:1 (w/v), 1 to 6:1 (w/v), 1 to 5:1 (w/v), 1 to 4:1 (w/v), 1 to 3:1 (w/v), 2 to 5:1 (w/v), 2 to 4:1 (w/v), or 2 to 3:1 (w/v), but is not limited thereto.

Preferably, in the composition according to the present invention, when the composition contains sugar, non-ionic surfactant, and stabilizer, the concentration ratio of the sugar, non-ionic surfactant, and stabilizer included in the composition may be 0.1 to 5:0.01 to 0.5:1 (w/v), 0.1 to 4.5:0.01 to 0.5:1 (w/v), 0.1 to 4:0.01 to 0.5:1 (w/v), 0.1 to 3.5:0.01 to 0.5:1 (w/v), 0.1 to 3:0.01 to 0.5:1 (w/v), 0.1 to 2.8:0.01 to 0.5:1 (w/v), 0.1 to 1:0.01 to 0.5:1 (w/v), 0.2 to 0.9:0.01 to 0.5:1 (w/v), 0.3 to 0.7:0.01 to 0.5:1 (w/v), 0.4 to 0.6:0.01 to 0.5:1 (w/v), 0.1 to 1:0.02 to 0.5:1 (w/v), 0.1 to 1:0.03 to 0.5:1 (w/v), 0.2 to 0.8:0.01 to 0.4:1 (w/v), 0.3 to 0.7:0.01 to 0.3:1 (w/v), or 0.4 to 0.6:0.03 to 0.3:1 (w/v), but is not limited thereto.

In one embodiment of the present invention, when the molecular weight of hyaluronic acid included in the liquid composition according to the present invention is 500,000 to 2,000,000 Da, the sugar may not be present or may be present at a concentration of 0.1 to 5% (w/v) based on the total composition; the non-ionic surfactant may be present at a concentration of 0.01 to 1% (w/v) based on the total composition; and the stabilizer may not be present or may be present at a concentration of 0.1 to 10% (w/v) based on the total composition, but is not limited thereto.

In another embodiment of the present invention, when the molecular weight of hyaluronic acid included in the liquid composition according to the present invention is 500,000 to 2,000,000 Da, the sugar may be present at a concentration of 0.1 to 5% (w/v) based on the total composition; the non-ionic surfactant may not be present or may be present at a concentration of 0.01 to 1% (w/v) based on the total composition; and the stabilizer may not be present or may be present at a concentration of 0.1 to 10% (w/v) based on the total composition, but is not limited thereto.

In yet another embodiment of the present invention, when the molecular weight of hyaluronic acid included in the liquid composition according to the present invention is 2,500,000 to 5,000,000 Da, the sugar may not be present or may be present at a concentration of 0.1 to 5% (w/v) based on the total composition; the non-ionic surfactant may be present at a concentration of 0.01 to 1% (w/v) based on the total composition; and the stabilizer may not be present or may be present at a concentration of 0.1 to 1% (w/v) based on the total composition, but is not limited thereto.

In the present invention, the composition may further include sodium phosphate as a buffering agent. The buffering agent may be present at a concentration of 25 to 100 mM, 25 to 90 mM, 25 to 80 mM, 25 to 70 mM, 25 to 60 mM, 30 to 100 mM, 30 to 90 mM, 30 to 80 mM, 30 to 70 mM, 30 to 60 mM, 40 to 100 mM, 40 to 90 mM, 40 to 80 mM, 40 to 70 mM, or 40 to 60 mM based on the total composition, but is not limited thereto.

In the present invention, the composition may be a ready-to-use injectable formulation, but is not limited thereto. The injectable formulation may further include one or more sugar alcohols selected from the group consisting of sorbitol, isomalt, erythritol, trehalose, maltitol, mannitol, xylitol, and mixtures thereof, but is not limited thereto and can be used without limitation if generally disclosed in the industry.

The pharmaceutical liquid composition of the present invention can be used for the treatment of neuromuscular-related disorders. That is, the present invention provides a pharmaceutical composition for the prevention or treatment of neuromuscular-related disorders, comprising the pharmaceutical liquid composition according to the present invention as an active ingredient.

In the present invention, the term "neuromuscular-related disorder" refers to a disorder induced by abnormalities in peripheral nerves and/or muscles. The neuromuscular-related disorder includes not only muscle tension abnormality disorders and non-muscle tension abnormality movement disorders (such as hemifacial spasm, tremor, rigidity), but also disorders caused by excessive secretion of glands regulated by the cholinergic nervous system. Preferably, the neuromuscular disorder may be characterized by hyperactive skeletal muscles.

Neuromuscular-related disorders according to the present invention can include, without limitation, any disorders known to exhibit therapeutic effects with conventional botulinum toxins. Non-limiting examples of such disorders include headache, migraine, tension headache, sinus headache, cervicogenic headache, hyperhidrosis, axillary hyperhidrosis, palmar hyperhidrosis, plantar hyperhidrosis, Frey syndrome, hyperkinetic skin lines, facial wrinkles, forehead wrinkles, crow's feet, perioral wrinkles, nasolabial wrinkles, skin disorders, acne, rhinitis, sinusitis, achalasia, strabismus, chronic anal fissure, blepharospasm, musculoskeletal pain, foot pain, plantar fasciitis, fibromyalgia, pancreatitis, tachycardia, prostatic hypertrophy, prostatitis, urinary retention, urinary incontinence, overactive bladder, hemifacial spasm, tremor, muscle spasms, gastrointestinal disorders, diabetes, gout, sialorrhea, detrusor-sphincter dyssynergia, post-stroke spasticity, wound healing, cerebral palsy in children, smooth muscle spasms, restenosis, focal dystonia, epilepsy, cervical dystonia, thyroid disorders, hypercalcemia, obsessive-compulsive disorder, osteoarthritis, temporomandibular joint disorder, Raynaud's syndrome, striae distensae, peritoneal adhesions, vasospasm, rhinorrhea, muscle contractures, laryngeal dystonia, writer's cramp, and carpal tunnel syndrome. The composition can be used for various purposes, such as treating the above-mentioned disorders and for cosmetic purposes.

In the present invention, the term "pharmaceutically acceptable salt" includes salts derived from pharmaceutically acceptable inorganic acids, organic acids, or bases. The term refers to a compound or composition that is suitable for contact with the tissue of a subject (for example, a human), having a reasonable benefit/risk ratio without excessive toxicity, irritation, allergic reactions, other problems or complications, and falling within the category of sound medical judgment.

Suitable acid examples include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methane sulfonic acid, formic acid, benzoic acid, malonic acid, gluconic acid, naphthalene-2-sulfonic acid, and benzene sulfonic acid. Acid addition salts can be prepared by conventional methods, for example, by dissolving the compound in an excess amount of an acidic aqueous solution, and precipitating the salt using a water-miscible organic solvent such as methanol, ethanol, acetone, or acetonitrile. Alternatively, acid or alcohol in an equimolar amount of the compound and water can be heated and subsequently evaporated to dry, or the precipitated salt can be obtained by suction filtration.

Salts derived from suitable bases may include alkali metals such as sodium and potassium, alkaline earth metals such as magnesium, and ammonium, but are not limited thereto. Alkali metal or alkaline earth metal salts can be obtained, for example, by dissolving the compound in an excess amount of an alkali metal hydroxide or alkaline earth metal hydroxide solution, filtering the insoluble compound salt, and evaporating and drying the filtrate. In this case, it is pharmaceutically suitable to prepare sodium, potassium, or calcium salts as metal salts, and the corresponding silver salts can be obtained by reacting the alkali metal or alkaline earth metal salt with an appropriate silver salt (e.g., silver nitrate).

Within the scope of any compound disclosed in the present invention, not only pharmaceutically acceptable salts, but also all isomers, hydrates, and solvates that can be prepared by conventional methods may be included.

Meanwhile, the composition according to the present invention may further include appropriate carriers, excipients, and diluents commonly used in the manufacture of pharmaceutical compositions. The excipients may include, for example, one or more selected from a group consisting of diluents, binders, disintegrants, lubricants, adsorbents, humectants, film-coating materials, and controlled-release additives.

The content of the liquid composition in the pharmaceutical composition of the present invention can be adjusted appropriately depending on the symptoms of the disease, the progress of the symptoms, and the patient's condition, for example, 0.0001 to 99.9 wt% or 0.001 to 50 wt% based on the total composition weight, but is not limited thereto. The content ratio is based on the dry weight after removing the solvent.

The pharmaceutical composition according to the present invention may further include appropriate carriers, excipients, and diluents commonly used in the manufacture of pharmaceutical compositions. The excipients may include, for example, one or more selected from a group consisting of diluents, binders, disintegrants, lubricants, adsorbents, humectants, film-coating materials, and controlled-release additives.

The pharmaceutical composition according to the present invention can be formulated and used in forms other than liquid formulations, such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols, etc. for external use. The external preparations may have formulations such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and Primojel^{®}; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro (OSI, OSIX, A, B, C, D, H, L), suppository base IV types (AB, B, A, BC, BBG, E, BGF, C, D, 299), suppostal (N, Es), Wecoby (W, R, S, M, Fs), and tegester triglyceride matter (TG-95, MA, 57) may be used.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Non-limiting examples of the non-aqueous solvent and the suspension include propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases.

As used herein, the "subject" refers to a subject in need of treatment of a disease, and more specifically, refers to a mammal such as a human or a non-human primate, a mouse, a rat, a dog, a cat, a horse, and a cow, but the present invention is not limited thereto.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "improvement" as used herein means all actions that reduce the degree of parameters related to a target disease, e.g., symptoms via administration of the composition according to the present invention.

The present invention also provides a cosmetic composition for the prevention or improvement of neuromuscular-related diseases, comprising the pharmaceutical liquid composition according to the present invention as an active ingredient.

A formulation for the cosmetic composition according to the present invention may include a skin lotion, a skin softener, a skin toner, an astringent, a lotion, a milk lotion, a moisturizing lotion, a nourishing lotion, a massage cream, a nourishing cream, a mist, a moisturizing cream, a hand cream, a hand lotion, a foundation, an essence, a nourishing essence, a pack, soap, a cleansing foam, a cleansing lotion, a cleansing cream, a cleansing oil, a cleansing balm, a body lotion or a body cleanser.

A cosmetic composition of the present invention may further include a composition selected from the group consisting of water-soluble vitamins, oil-soluble vitamins, polymer peptides, polymeric polysaccharides, and sphingolipids.

In the cosmetic composition of the present invention, other ingredients that are conventionally blended in cosmetics may be combined, in addition to the essential components, as needed.

Examples of additional ingredients to be mixed may include lipid components, a humectant, an emollient, a surfactant, organic and inorganic pigments, organic powder, a UV absorbent, a preservative, a sanitizer, an antioxidant, a plant extract, a pH adjuster, alcohol, pigments, flavors, a blood circulation promoter, a cooling agent, an anti-diaphoretic, and purified water.

The lipid components may include, for example, ester lipids, hydrocarbon lipids, silicone lipids, fluorine lipids, animal fats, vegetable oil, or the like.

The ester lipids may include, for example, glyceryl tri 2-ethylhexanoate, cetyl 2-ethylhexanoate, isopropyl myristate, butyl myristate, isopropyl palmitate, ethyl stearate, octyl palmitate, isocetyl isostearate, butyl stearate, ethyl linolate, isopropyl linolate, ethyl oleate, isocetyl myristate, isostearyl myristate, isostearyl palmitate, octyldodecyl myristate, isocetyl isostearate, diethyl sebacate, diisopropyl adipate, isoalkyl neopentanate, tri(capryl, capric acid)glyceryl, trimethylolpropane tri 2-ethylhexanoate, trimethylolpropane triisostearate, pentaerythritol tetra 2-ethylhexanoate, cetyl caprylate, decyl laurate, hexyl laurate, decyl myristate, myristyl myristate, cetyl myristate, stearyl stearate, decyl oleate, cetyl ricinoleate, isostearyl laurate, isotridecyl myristate, isocetyl palmitate, octyl stearate, isocetyl stearate, isodecyl oleate, octyldodecyl oleate, octyldodecyl linolate, isopropyl isostearate, cetostearyl 2-ethylhexanoate, stearyl 2-ethylhexanoate, hexyl isostearate, ethyleneglycol dioctanoate, ethyleneglycol dioleate, propyleneglycol dicaprinate, propyleneglycol di(caprylate, caprinate), propyleneglycol dicaprylate, neopentylglycol dicaprinate, neopentylglycol dioctanoate, glyceryl tricaprylate, glyceryl triundecylate, glyceryl triisopalmitate, glyceryl triisostearate, octyldodecyl neopentanoate, isostearyl octanoate, octyl isononanoate, hexyldecyl neodecanoate, octyldodecyl neodecanoate, isocetyl isostearate, isostearyl isostearate, octyldecyl isostearate, polyglycerin ester oleate, polyglycerin ester isostearate, triisocetyl citrate, triisoalkyl citrate, triisooctyl citrate, lauryl lactate, myristyl lactate, cetyl lactate, octyldecyl lactate, triethyl citrate, acetyltriethyl citrate, acetyltributyl citrate, trioctyl citrate, diisostearyl malate, 2-ethylhexyl hydroxystearate, di 2-ethylhexyl succinate, diisobutyl adipate, diisopropyl sebacate, dioctyl sebacate, cholesteryl stearate, cholesteryl isostearate, cholesteryl hydroxystearate, cholesteryl oleate, dihydrocholesteryl oleate, phytosteryl isostearate, phytosteryl oleate, isocetyl 12-stearoyl hydroxystearate, stearoyl 12-stearoyl hydroxystearate, isostearyl 12-stearoyl hydroxystearate, and the like.

The hydrocarbon lipids may include, for example, squalene, liquid paraffin, alpha-olefin oligomers, isoparaffin, ceresine, paraffin, liquid isoparaffin, polybutene, microcrystalline wax, Vaseline, and the like.

The silicone lipids may include, for example, polymethyl silicon, methylphenyl silicon, methyl cyclopolysiloxane, octamethyl polysiloxane, decamethyl polysiloxane, dodecamethyl cyclosiloxane, dimethylsiloxane/ methylcetyloxysiloxane copolymers, dimethylsiloxane/ methylstearoxysiloxane copolymers, alkyl-modified silicon oil, amino-modified silicon oil, and the like.

The fluorine lipids may include perfluoropolyether and the like.

The animal or vegetable oil may include avocado oil, almond oil, olive oil, sesame oil, rice bran oil, safflower oil, soybean oil, corn oil, rape flower oil, apricot kernel oil, palm kernel oil, palm oil, castor oil, sunflower oil, grape seed oil, cotton seed oil, coconut oil, tallow nut oil, wheat germ oil, rice germ oil, Shea butter, evening primrose oil, macadamia nut oil, meadow foam seed oil, yolk oil, beef tallow, hemp seed oil, mink oil, orange roughy oil, jojoba oil, candelilla wax, carnauba wax, liquid lanolin, dehydrated castor oil, and the like.

The humectant may include water-soluble low molecular humectants, oil-soluble molecular humectants, water-soluble polymers, oil-soluble polymers, and the like.

The water-soluble low molecular humectants may include serine, glutamine, sorbitol, mannitol, pyrrolidone-sodium carboxylate, glycerin, propylene glycol, 1,3-butylene glycol, ethylene glycol, polyethylene glycol B (degree of polymerization: n=2 or higher), polypropylene glycol (degree of polymerization: n=2 or higher), polyglycerin B (degree of polymerization: n=2 or higher), lactic acid, lactates, and the like.

The oil-soluble low molecular humectants may include cholesterol, cholesterol ester, and the like.

The water-soluble polymers may include carboxyvinyl polymers, polyasparaginic acid salts, tragacanth, xanthan gum, methyl cellulose, hydroxymethyl cellulose, hydroxylethyl cellulose, hydroxypropyl cellulose, carboxymethyl cellulose, water-soluble chitin, chitosan, dextrin, and the like.

The oil-soluble polymers may include, for example, polyvinyl pyrrolidone/eicosen copolymers, polyvinyl pyrrolidone/hexadecene copolymers, nitrocellulose, dextrin fatty acid ester, silicone polymers, and the like.

The emollients may include, for example, long chain cholesterylester acyl glutamate, cholesteryl hydroxystearate, 12-hydroxystearic acid, stearic acid, rosin acid, lanolin fatty acid cholesteryl ester, and the like.

The surfactants may include, for example, non-ionic surfactants, anionic surfactants, cationic surfactants, amphoteric surfactants, and the like.

The non-ionic surfactants may include self-emulsion type glycerin monostearate, propyleneglycol fatty acid ester, glycerin fatty acid ester, polyglycerin fatty acid ester, sorbitan fatty acid ester, polyoxyethylene (POE) sorbitan fatty acid ester, POE sorbit fatty acid ester, POE glycerin fatty acid ester, POE alkylethers, POE fatty acid ester, POE dehydrated castor oil, POE castor oil, polyoxyethylene/polyoxypropylene (POE/POP) copolymers, POE/POP alkylethers, polyether-modified silicone, alkanolamide laurate, alkylamine oxide, hydrated soy phospholipids, and the like.

The anionic surfactants may include fatty acid soap, α-acylsulfonate, alkyl sulfonates, alkylallyl sulfonates, alkylnaphthalene sulfonates, alkyl sulfates, POE alkylether sulfates, alkylamide sulfates, alkyl phosphates, POE alkyl phosphates, alkylamide phosphates, alkyloyl alkyltaurin salts, N-acylamino acid salts, POE alkylether carboxylates, alkyl sulfosuccinates, sodium alkyl sulfoacetates, acylated hydrolyzed collagen peptide salts, perfluoroalkyl ester phosphates, and the like.

The cationic surfactants may include, for example, alkyltrimethylammonium chloride, stearyltrimethylammonium chloride, steraryltrimethylammonium bromide, cetostearyl trimethylammonium chloride, distearyl dimethylammonium chloride, stearylaryl dimethylbenzylammonium chloride, behenyltrimethylammonium bromide, benzalkonium chloride, diethylaminoethylamide stearate, dimethylaminopropylamide stearate, quaternary ammonium salts of lanolin derivatives, and the like.

The amphoteric surfactants may include carboxybetaine, amidebetaine, sulfobetaine, hydroxysulfobetaine, amidesulfobetaine, phosphobetaine, aminocarboxylate, imidazoline derivatives, amideamine-based amphoteric surfactants, and the like.

The organic and inorganic pigments may include: inorganic pigments such as silicic acid, anhydrous silicic acid, magnesium silicate, talc, sericite, mica, kaolin, bengala, clay, bentonite, titanium dioxide-coated mica, bismuth oxychloride, zirconium oxide, magnesium oxide, zinc oxide, titanium oxide, aluminum oxide, calcium sulfate, barium sulfate, magnesium sulfate, calcium carbonate, magnesium carbonate, iron oxide, ultramarine, chromium oxide, chromium hydroxide, calamine, and combinations thereof; organic pigments such as polyamide, polyester, polypropylene, polystyrene, polyurethane, vinyl resin, urea resin, phenol resin, fluorine resin, silicon resin, acryl resin, melamine resin, epoxy resin, polycarbonate resin, divinyl benzene/styrene copolymers, silk powder, cellulose, CI pigment yellow, and CI pigment orange; and composite pigments of inorganic and organic pigments.

The organic powder may include: metallic soap such as calcium stearate; metal salts of alkyl phosphoric acid such as zinc sodium cetylate, zinc laurylate, and calcium laurylate; polymetallic salts of acylamino acid such as calcium N-lauroyl-beta-alanine, zinc N-lauroyl-beta-alanine, and calcium N-lauroylglycine; polymetallic salts of amide sulfonates such as calcium N-lauroyl-taurine and calcium N-palmitoyl-taurine.; N-acyl alkaline amino acids such as N-epsilon-lauroyl-L-lysine, N-epsilon-palmitoyl lysine, N-α-palmitoylol nitin, N-α-lauroyl arginine, and N-α-dehydrated tallow fatty acid acyl arginine; N-acyl polypeptides such as N-lauroyl glycylglycine; α-amino fatty acids such as α-aminocaprylic acid and α-aminolauric acid; polyethylene; polypropylene; nylon; polymethylmethacrylate; polystyrene; divinylbenzene/styrene copolymers; ethylene tetrafluoride; and the like.

The UV absorbents may include para-aminobenzoic acid, ethyl para-aminobenzoate, amyl para-aminobenzoate, octyl para-aminobenzoate, ethyleneglycol salicylate, phenyl salicylate, octyl salcylate, benzyl salicylate, butylphenyl salicylate, homomentyl salicylate, benzyl cinnamate, para-methoxycinnamic acid-2-ethoxylethyl, octyl paramethoxycinnamate, mono-2-ethylhexaneglyceryl diparamethoxycinnamate, isopropyl paramethoxycinnamate, diisopropyl/diisopropyl cinnamic acid ester mixtures, urocanic acid, ethyl urocanate, hydroxymethoxybenzophenone, hydroxymethoxybenzophenone sulfonic acid and salts thereof, dihydroxymethoxybenzophenone, sodium dihydroxymethoxybenzophenone disulfonate, dihydroxybenzophenone, tetrahydroxybenzophenone, 4-tert-butyl-4'-methoxydibenzoylmethane, 2,4,6-trianilino-p-(carbo-2'-ethylhexyl-1'-oxy)-1,3,5-triazine, 2-(2-hydroxy-5-methylphenyl)benzotriazole, and the like.

The sanitizers may include hinokitiol, trichloric acid, trichlorohydroxydiphenylether, chlorohexidine gluconate, phenoxyethanol, resorcine, isopropylmethylphenol, azulene, salicylic acid, zinc pyrithione, benzalkonium chloride, light sensitive element No. 301, sodium mononitroguaiacol, undecylenic acid, and the like.

The antioxidants may include butylhydroxyanisole, propyl gallate, elisorbic acid, and the like.

The pH adjusters may include citric acid, sodium citrate, malic acid, sodium malate, fumaric acid, sodium fumarate, succinic acid, sodium succinate, sodium hydroxide, sodium monohydrophosphate, and the like.

The alcohols may include higher alcohols such as cetyl alcohol.

In addition, additional ingredients to be mixed are not limited to the above examples, and any one of the above ingredients may be mixed within a range that does not adversely affect the objectives and effects of the present invention, but may range from 0.01 wt% to 5 wt% or 0.01 wt% to 3 wt% with respect to the total weight of the composition.

For lotion, paste, cream, or gel preparations of the present invention, as a carrier ingredient, animal fiber, vegetable fiber, wax, paraffin, starch, tragacanth, a cellulose derivative, polyethylene glycol, silicon, bentonite, silica, talc, zinc oxide, or the like may be used.

For powder or spray preparations of the present invention, lactose, talc, silica, aluminum hydroxide, calcium silicate, or polyamide powder may be used as a carrier ingredient. In particular, in the case of spray preparations, the composition may further include a propellant such as chlorofluorohydrocarbon, propane/butane, or dimethyl ether.

For solution or emulsion preparations of the present invention, a solvent, a solubilizing agent, or an emulsifying agent may be used as a carrier ingredient, and the carrier ingredient may be, for example, water, ethanol, isopropanol, ethyl carbonate, ethyl acetate, benzyl alcohol, benzyl benzoate, propylene glycol, 1,3-butyl glycol oil, a glycerol aliphatic ester, polyethylene glycol, or a sorbitan fatty acid ester.

For suspension preparations of the present invention, as a carrier ingredient, a liquid diluent such as water, ethanol, or propylene glycol, a suspending agent such as ethoxylated isostearyl alcohol, polyoxyethylene sorbitol ester, or polyoxyethylene sorbitan ester, micro-crystalline cellulose, aluminum methahydroxide, bentonite, agar, tragacanth, or the like may be used.

For surfactant-containing cleansing preparations of the present invention, as a carrier ingredient, an aliphatic alcohol sulfate, an aliphatic alcohol ether sulfate, a sulfosuccinate monoester, isethionate, imidazolinium derivatives, methyltaurate, sarcosinate, fatty acid amide ether sulfate, alkylamidobetaine, an aliphatic alcohol, a fatty acid glyceride, a fatty acid diethanol amide, vegetable oil, a lanolin derivative, an ethoxylated glycerol fatty acid ester, or the like may be used.

The terms and words used in this specification and claims should not be interpreted in a conventional or dictionary sense but should be interpreted in the meaning and concept that is consistent with the technical idea of the present invention, based on the principle that the inventor can appropriately define the concepts of terms to best describe their invention.

Hereinafter, preferable embodiments are presented to aid in understanding the present invention. However, the following embodiments are provided only for a better understanding of the invention, and the scope of the present invention is not limited by these embodiments.

### [Examples]

### Experimental Materials and Methods

### 1. Preparation of Botulinum Toxin Liquid Composition

To evaluate the biological activity retention of botulinum toxin depending on the type and concentration of additives, botulinum toxin liquid formulations containing hyaluronic acid and additives such as sugars, non-ionic surfactants, stabilizers, or their combinations were prepared. Specifically, hyaluronic acid (molecular weight of hyaluronic acid used in Examples 1 to 4 = 1 MDa, molecular weight of hyaluronic acid used in Example 5 = 3 MDa) was dissolved in 0.9% (w/v) sodium chloride solution to a concentration of 10 mg/mL. Next, additive candidate materials were dissolved individually or in a combination of two or more additives in a 50 mM sodium phosphate buffer (pH 6.0), and added to the hyaluronic acid solution at 10% (v/v). Botulinum toxin was added to the prepared hyaluronic acid-additive mixture to a final concentration of 20 units/mL, followed by stirring and mixing for 1 hour. The botulinum toxin liquid composition prepared as described above was stored at 4°C.

### 2. Animal Potency Test 1 - Stability Evaluation of Botulinum Toxin Depending on Additives

The animal potency test was conducted by injecting 0.1 mL of liquid compositions of the present invention, prepared with various concentrations of diluted additives individually or in a combination of two or more additives, into the abdominal cavity of ICR-mice (5 weeks old, weight: 19-22 g, 10 mice per group), and the number of dead mice was observed for 3 days. A statistically significant retention of biological activity was evaluated for groups that showed a mortality rate of 70% or more. Considering the sampling error due to the viscosity of the composition and other experimental errors, groups with mortality rates below the standard were subjected to additional confirmation tests in the next cycle, and groups with mortality rates above the standard continued with the tests. Other animal potency test results in the present invention were performed in the same manner as described above.

### 3. Animal Potency Test 2 - Calculation of Potency and Specific Potency

Liquid compositions of the present invention, prepared with various concentrations of diluted additives individually or in a combination of two or more additives, were injected into the abdominal cavity of ICR-mice (5 weeks old, weight: 19-22 g, 10 mice per group) at 0.1 mL per mouse, and the number of dead mice was observed for 3 days. The activity of botulinum toxin was calculated using a statistical program (Combistats, version 4.0, EDQM) based on the obtained data.

### Example 1. Confirmation of Low-temperature Stability of Botulinum Toxin Liquid Composition Containing Saccharide

A screening experiment was conducted for various types of sugars excluding the sugar used as an additive in conventional botulinum toxin liquid formulations, and lactose monohydrate and fructose were selected as the final candidates. Animal potency test 1, as described in Chapter 2 of the experimental materials and methods, was performed after adding lactose monohydrate or fructose at different amounts.

The results are shown in Table 1 and FIG. 1 below. The low-temperature stability of the test groups containing 0.1% (w/v) lactose monohydrate and 0.5% (w/v) fructose was significantly improved compared to other test groups, and the biological activity of botulinum toxin in these two test groups was maintained until the 10th week. The test results were reflected in determining the mixing concentration of each additive in the comparative stabilization efficacy test with multiple additives.

**[Table 1]**

| Additives | | Number of deaths / Total number of individuals | | | |
|---|---|---|---|---|---|
| Group | Content (% (w/v)) | Week 0 | Week 5 | Week 10 | Week 15 |
| Lactose Monohydrate | 0.1 | 9/10 | 7/10 | 6/10 | 0/10 |
| | 1 | 8/10 | 0/10 | 0/10 | 0/10 |
| Fructose | 0.5 | 9/10 | 9/10 | 8/10 | 0/10 |
| | 5 | 8/10 | 6/10 | 1/10 | 0/10 |

### Example 2. Low-temperature Stability Verification of a Botulinum Toxin Liquid Composition Containing a Non-ionic Surfactant

Screening experiments were performed on various types of non-ionic surfactants, excluding the non-ionic surfactants that are being used as additives in conventional botulinum toxin liquid formulations, including surfactants containing non-ionic surfactants. As a result, polyoxyethylene (40) hydrogenated castor oil (referred to as 'HCO-40') was selected as the final candidate. Subsequently, animal potency test 1 described in section 2 of the experimental materials and methods was conducted after adding HCO-40 in different concentrations.

As shown in Table 2 and FIG. 2 below, the test group containing 0.2% (w/v) HCO-40 showed low-temperature stability up to 15 weeks. The results of the test were used to determine the concentration of each additive in the stability comparison test when multiple additives were mixed together.

**[Table 2]**

| Additives | | Number of deaths / Total number of individuals | | | | |
|---|---|---|---|---|---|---|
| Group | Content (% (w/v)) | Week 0 | Week 5 | Week 10 | Week 12 | Week 15 |
| Control | 0 | 9/10 | 8/10 | 0/10 | - | - |
| HCO-40 | 0.05 | 8/10 | 7/10 | 6/10 | 8/10 | 1/10 |
| | 0.2 | 9/10 | 9/10 | 6/10 | 6/10 | 5/10 |

### Example 3. Low-temperature Stability Verification of a Botulinum Toxin Liquid Composition Containing a Stabilizer

Screening experiments were conducted on various types of stabilizers, excluding the stabilizers that are being used as additives in conventional botulinum toxin liquid formulations, and as a result, glycerin was selected as the final candidate. Subsequently, animal potency test 1 described in section 2 of the experimental materials and methods was conducted after adding glycerin in different concentrations.

As shown in Table 3 and FIG. 3 below, the test group containing 1% (w/v) glycerin showed low-temperature stability up to 25 weeks. The results of the test were used to determine the concentration of each additive in the stability comparison test when multiple additives were mixed together.

**[Table 3]**

| Additives | | Number of deaths / Total number of individuals | | | | | | |
|---|---|---|---|---|---|---|---|---|
| Group | Content (% (w/v)) | Week 0 | Week 5 | Week 10 | Week 15 | Week 17 | Week 20 | Week 25 |
| Control | 0 | 9/10 | 8/10 | 0/10 | - | - | - | - |
| Glycerin | 0.1 | 10/10 | 6/10 | 0/10 | - | - | - | - |
| | 1.0 | 9/10 | 9/10 | 8/10 | 7/10 | 8/10 | 8/10 | 10/10 |
| | 5.0 | 3/10 | - | - | - | - | - | - |

### Example 4. Stability Comparison Verification of a Botulinum Toxin Liquid Composition (Using 1 MDa Hyaluronic Acid) Containing Multiple Additives

Referring to the animal test results of the individual additives performed in Examples 1 to 3, each additive was mixed in various combinations, and the stability was verified. However, in the case of fructose, a concentration of 2.5% (w/v) was added, which was the median value of the concentration used in the test group where it was added individually. A sample without any additives was used as a negative control. Periodic animal testing was conducted on the test group that met the criteria for maintaining the stability of the set botulinum toxin. The composition of the entire test group is shown in the table below.

### [Composition of the Test Group]

| Test Group | Composition |
|---|---|
| 1 | HCO-40 0.2% (w/v) |
| 2 | Fructose 0.5% (w/v) + HCO-40 0.2% (w/v) |
| 3 | HCO-40 0.2% (w/v) + Glycerin 1% (w/v) |
| 4 | Fructose 0.5% (w/v) + HCO-40 0.2% (w/v) + Glycerin 1% (w/v) |
| 5 | Fructose 0.5% (w/v) + HCO-40 0.05% (w/v) |
| 6 | HCO-40 0.05% (w/v) + Glycerin 1% (w/v) |
| 7 | Fructose 0.5% (w/v) + HCO-40 0.05% (w/v) + Glycerin 1% (w/v) |
| 8 | Fructose 2.5% (w/v) |
| 9 | Fructose 2.5% (w/v) + HCO-40 0.2% (w/v) |
| 10 | Fructose 2.5% (w/v) + Glycerin 1% (w/v) |
| 11 | Fructose 2.5% (w/v) + HCO-40 0.2% (w/v) + Glycerin 1% (w/v) |

The results are shown in Table 4 and FIG. 4 below. As a result of confirming the storage stability for 52 weeks, the test groups with additives mixed showed a higher average mortality rate compared to the test groups with additives added individually.

In particular, according to the mouse mortality rates measured during the periods of 0, 3, 6, 10, 15, 20, 25, 36, and 52 weeks, it was found that the botulinum toxin in test group 1 [containing HCO-40 0.2% (w/v)], test group 2 [containing fructose 0.5% (w/v), HCO-40 0.2% (w/v)], test group 4 [containing fructose 0.5% (w/v), HCO-40 0.2% (w/v), glycerin 1% (w/v)], and test group 7 [containing fructose 0.5% (w/v), HCO-40 0.05% (w/v), glycerin 1% (w/v)] satisfied the stability criterion (showing a mortality rate of 70% or higher for all observation periods up to 20 weeks). In particular, it was confirmed that test group 3 [containing HCO-40 0.2% (w/v), glycerin 1% (w/v)] and test group 4 [containing fructose 0.5% (w/v), HCO-40 0.2% (w/v), glycerin 1% (w/v)] maintained high mortality rates of 80% or more even in the 52nd week, thus achieving long-term stability of botulinum toxin for more than 12 months when additives that meet the above composition are mixed.

**[Table 4]**

| Group | Additive concentration (% (w/v)) | | | Number of deaths / total number of individuals according to the week | | | | | | | | |
|---|---|---|---|---|---|---|---|---|---|---|---|---|
| | Fructose | HCO-40 | Glycerin | 0 | 3 | 6 | 10 | 15 | 20 | 25 | 36 | 52 |
| Contr ol | 0 | 0 | 0 | 9/10 | 8/10 | 0/10 | | | | | | |
| Test 1 | 0 | 0.2 | 0 | 8/10 | 7/10 | 6/10 | 8/10 | 9/10 | 8/10 | 5/10 | 8/10 | 1/10 |
| Test 2 | 0.5 | 0.2 | 0 | 8/10 | 10/10 | 7/10 | 9/10 | 9/10 | 8/10 | 5/10 | 0/10 | 0/10 |
| Test 3 | 0 | 0.2 | 1.0 | 10/10 | 6/10 | 7/10 | 8/10 | 7/10 | 8/10 | 5/10 | 9/10 | 10/10 |
| Test 4 | 0.5 | 0.2 | 1.0 | 9/10 | 7/10 | 10/10 | 7/10 | 10/1 0 | 7/10 | 5/10 | 8/10 | 9/10 |
| Test 5 | 0.5 | 0.05 | 0 | 7/10 | 6/10 | 7/10 | 10/10 | 7/10 | 6/10 | 7/10 | 4/10 | 0/10 |
| Test 6 | 0 | 0.05 | 1.0 | 8/10 | 5/10 | 4/10 | 6/10 | 6/10 | 7/10 | 5/10 | 4/10 | 5/10 |
| Test 7 | 0.5 | 0.05 | 1.0 | 8/10 | 8/10 | 9/10 | 8/10 | 7/10 | 7/10 | 10/10 | 10/10 | 3/10 |
| Test 8 | 2.5 | 0 | 0 | 9/10 | 8/10 | 7/10 | 8/10 | 6/10 | 9/10 | 9/10 | 0/10 | 0/10 |
| Test 9 | 2.5 | 0.2 | 0 | 8/10 | 7/10 | 10/10 | 10/10 | 5/10 | 3/10 | 1/10 | 0/10 | 0/10 |
| Test 10 | 2.5 | 0 | 1.0 | 8/10 | 6/10 | 10/10 | 10/10 | 7/10 | 7/10 | 6/10 | 1/10 | 0/10 |
| Test 11 | 2.5 | 0.2 | 1.0 | 6/10 | 9/10 | 9/10 | 7/10 | 9/10 | 6/10 | 7/10 | 5/10 | 3/10 |

### Example 5. Comparison of Stabilization Efficacy of Botulinum Toxin Liquid Compositions Containing Multiple Additives (Using 3 MPa Hyaluronic Acid)

Referring to the results of animal tests performed with the individual addition of additive candidates in Examples 1 to 3, the stabilization efficacy was confirmed after mixing each additive in various combinations. Exceptionally for fructose, 2.5% (w/v) was added, which is the median concentration used in the test group with individual addition. A sample without any additives was used as a negative control. Periodic animal tests were conducted for test groups meeting the criteria for maintaining the stability of the botulinum toxin. Experiments were conducted for test groups 1 to 11, and the composition of additives in each test group is the same as in the additive composition table of Example 4.

The results are presented in Table 5 and FIG. 5. After verifying storage stability for 52 weeks, test groups with mixed additives showed a higher average mortality rate than test groups with individual additives.

In particular, botulinum toxin in test group 1 [containing 0.2% (w/v) HCO-40], test group 4 [containing 0.5% (w/v) fructose, 0.2% (w/v) HCO-40, and 1% (w/v) glycerin], and test group 6 [containing 0.05% (w/v) HCO-40 and 1% (w/v) glycerin] not only met the stability criteria (showing a mortality rate of 70% or more during all observation periods) but also demonstrated a high mortality rate of 80% or more at week 52, confirming that the long-term stability effect of botulinum toxin lasting for 12 months or more can be achieved when the additives satisfying the above composition are mixed.

**[Table 5]**

| Group | Additive concentration (% (w/v)) | | | Number of deaths / total number of individuals according to the week | | | | | | | | |
|---|---|---|---|---|---|---|---|---|---|---|---|---|
| | Fructose | HCO-40 | Glycerin | 0 | 3 | 6 | 10 | 15 | 20 | 25 | 36 | 52 |
| Control | 0 | 0 | 0 | 9/10 | 8/10 | 0/10 | | | | | | |
| Test 1 | 0 | 0.2 | 0 | 7/10 | 9/10 | 9/10 | 9/10 | 8/10 | 10/10 | 8/10 | 5/10 | 9/10 |
| Test 2 | 0.5 | 0.2 | 0 | 10/10 | 7/10 | 6/10 | 9/10 | 6/10 | 7/10 | 0/10 | 0/10 | 0/10 |
| Test 3 | 0 | 0.2 | 1.0 | 8/10 | 6/10 | 8/10 | 10/10 | 7/10 | 10/10 | 8/10 | 8/10 | 5/10 |
| Test 4 | 0.5 | 0.2 | 1.0 | 9/10 | 9/10 | 9/10 | 7/10 | 9/10 | 7/10 | 8/10 | 4/10 | 9/10 |
| Test 5 | 0.5 | 0.05 | 0 | 10/10 | 8/10 | 10/10 | 5/10 | 7/10 | 5/10 | 6/10 | 0/10 | 0/10 |
| Test 6 | 0 | 0.05 | 1.0 | 9/10 | 7/10 | 10/10 | 7/10 | 8/10 | 7/10 | 10/10 | 9/10 | 10/10 |
| Test 7 | 0.5 | 0.05 | 1.0 | 10/10 | 8/10 | 10/10 | 6/10 | 8/10 | 6/10 | 6/10 | 6/10 | 4/10 |
| Test 8 | 2.5 | 0 | 0 | 8/10 | 9/10 | 9/10 | 7/10 | 4/10 | 7/10 | 0/10 | 0/10 | 0/10 |
| Test 9 | 2.5 | 0.2 | 0 | 4/10 | 9/10 | 7/10 | 7/10 | 7/10 | 10/10 | 5/10 | 0/10 | 0/10 |
| Test 10 | 2.5 | 0 | 1.0 | 9/10 | 8/10 | 8/10 | 7/10 | 6/10 | 7/10 | 2/10 | 0/10 | 0/10 |
| Test 11 | 2.5 | 0.2 | 1.0 | 9/10 | 8/10 | 10/10 | 6/10 | 6/10 | 10/10 | 9/10 | 0/10 | 0/10 |

| | | | | | | | | | | | | |
|---|---|---|---|---|---|---|---|---|---|---|---|---|
| * The additive compositions of Test Group 1 through Test Group 11 are identical to those in Table 4. | | | | | | | | | | | | |

The description of the present invention as described above is for illustrative purposes only, and those of ordinary skill in the technical field to which the invention pertains will understand that the technical idea or essential features of the invention can be easily transformed into other specific forms without changing. Therefore, the examples described above should be understood as being exemplary in all respects and not limiting.

### [Industrial Applicability]

The present invention can significantly increase the stability during long-term storage of botulinum toxin by providing a liquid composition that includes an additive for maintaining the pharmacological activity of the active ingredients, botulinum toxin, and hyaluronic acid. By combining pH regulators, sugars, surfactants, stabilizers, and other additives at specific concentrations and adding them to the botulinum toxin liquid formulation, it demonstrates excellent effects in preventing physical and chemical instability and the denaturation of the tertiary structure of the botulinum toxin. Thus, the use of the composition according to the present invention can expand the application forms of existing botulinum toxin, which were limited to dry formulations, to liquid formulations, and thus various types of products can be developed. Therefore, it is expected to be utilized in various industrial fields.

Furthermore, the increased stability of botulinum toxin in the liquid state utilizing the composition of the present invention can guarantee stability during the formulation process of botulinum toxin, and it can be applied to controlled-release injectable drug formulations in sustained-release or immediate-release forms, or can be utilized in the development of transdermal drugs using microstructures such as microneedle patches. In addition, the active ingredient, hyaluronic acid, which is excellent in biocompatibility, can provide an additional anti-inflammatory effect and confer viscosity within the final product, thereby expecting localized therapeutic effects of botulinum toxin. Therefore, the composition of the present invention is expected to be useful in various therapeutic fields where botulinum toxin can be applied.

## Claims

1. A pharmaceutical liquid composition comprising the following (i) to (iii) as active ingredients:
(i) botulinum toxin;
(ii) hyaluronic acid or a pharmaceutically acceptable salt thereof; and
(iii) one or more additives selected from the group consisting of sugars, non-ionic surfactants, and stabilizers.

2. The pharmaceutical liquid composition of claim 1, wherein the composition comprises botulinum toxin at a concentration of 5 to 40 units/mL based on the total composition.

3. The pharmaceutical liquid composition of claim 1, wherein the botulinum toxin is in a form that does not contain a complexing protein or is in a complex form containing a complexing protein.

4. The pharmaceutical liquid composition of claim 1, wherein the hyaluronic acid has a molecular weight of 30,000 to 5,000,000 Daltons (Da).

5. The pharmaceutical liquid composition of claim 1, wherein the composition comprises hyaluronic acid at a concentration of 5 to 250 mg/mL based on the total composition.

6. The pharmaceutical liquid composition of claim 1, wherein the sugar is lactose monohydrate or fructose.

7. The pharmaceutical liquid composition of claim 1, wherein the composition comprises the sugar at a concentration of 0.01 to 5 % (w/v) based on the total composition.

8. The pharmaceutical liquid composition of claim 1, wherein the non-ionic surfactant is polyoxyethylene hydrogenated castor oil.

9. The pharmaceutical liquid composition of claim 8, wherein the polyoxyethylene hydrogenated castor oil has a molar ratio of ethylene oxide of 10 to 60.

10. The pharmaceutical liquid composition of claim 1, wherein the composition comprises the non-ionic surfactant at a concentration of 0.01 to 1 % (w/v) based on the total composition.

11. The pharmaceutical liquid composition of claim 1, wherein the stabilizer is glycerin.

12. The pharmaceutical liquid composition of claim 1, wherein the composition comprises the stabilizer at a concentration of 0.1 to 10 % (w/v) based on the total composition.

13. The pharmaceutical liquid composition of claim 1, wherein:
the hyaluronic acid has a molecular weight of 500,000 to 2,000,000 Daltons;
the sugar is either not comprised or is comprised at a concentration of 0.1 to 5 % (w/v) based on the total composition;
the non-ionic surfactant is comprised at a concentration of 0.01 to 1% (w/v) based on the total composition; and
the stabilizer is either not comprised or is comprised at a concentration of 0.1 to 10% (w/v) based on the total composition.

14. The pharmaceutical liquid composition of claim 1, wherein:
the hyaluronic acid has a molecular weight of between 500,000 to 2,000,000 Daltons;
the sugar is comprised at a concentration of 0.1 to 5% (w/v) based on the total composition;
the non-ionic surfactant is either not comprised or is comprised at a concentration of 0.01 to 1% (w/v) based on the total composition; and
the stabilizer is either not comprised or is comprised at a concentration of 0.1 to 10% (w/v) based on the total composition.

15. The pharmaceutical liquid composition of claim 1, wherein:
the hyaluronic acid has a molecular weight of between 2,500,000 to 5,000,000 Daltons;
the sugar is either not comprised or is comprised at a concentration of 0.1 to 5% (w/v) based on the total composition;
the non-ionic surfactant is comprised at a concentration of 0.01 to 1% (w/v) based on the total composition; and
the stabilizer is either not comprised or is comprised at a concentration of 0.1 to 1% (w/v) based on the total composition.

16. The pharmaceutical liquid composition of claim 1, wherein the composition further comprises sodium phosphate as a buffer at a concentration of 25 to 100 mM.

17. The pharmaceutical liquid composition of claim 1, wherein the composition is a ready-to-use injectable formulation.

18. A method of maintaining the biological activity of botulinum toxin, utilizing a pharmaceutical liquid composition comprising the following (i) to (iii) as active ingredients:
(i) botulinum toxin;
(ii) hyaluronic acid or its pharmaceutically acceptable salt; and
(iii) one or more additives selected from the group consisting of sugar, non-ionic surfactant, and stabilizer.

19. A method for manufacturing a pharmaceutical liquid composition for long-term storage of botulinum toxin, comprising the step of mixing botulinum toxin; hyaluronic acid or its pharmaceutically acceptable salt; and one or more additives selected from the group consisting of sugars, non-ionic surfactants, and stabilizers.

20. A pharmaceutical composition for preventing or treating of neuromuscular related disease, comprising the pharmaceutical liquid composition of Claim 1 as an active ingredient.

21. The pharmaceutical composition of claim 20, wherein the neuromuscular related disease is one or more selected from the group consisting of headache, migraine, tension headache, sinus headache, cervicogenic headache, hyperhidrosis, axillary hyperhidrosis, palmar hyperhidrosis, plantar hyperhidrosis, Frey syndrome, hyperkinetic skin lines, facial wrinkles, forehead wrinkles, crow's feet, perioral wrinkles, nasolabial wrinkles, skin disorders, acne, rhinitis, sinusitis, achalasia, strabismus, chronic anal fissure, blepharospasm, musculoskeletal pain, foot pain, plantar fasciitis, fibromyalgia, pancreatitis, tachycardia, prostatic hypertrophy, prostatitis, urinary retention, urinary incontinence, overactive bladder, hemifacial spasm, tremor, muscle spasms, gastrointestinal disorders, diabetes, gout, sialorrhea, detrusor-sphincter dyssynergia, post-stroke spasticity, wound healing, cerebral palsy in children, smooth muscle spasms, restenosis, focal dystonia, epilepsy, cervical dystonia, thyroid disorders, hypercalcemia, obsessive-compulsive disorder, osteoarthritis, temporomandibular joint disorder, Raynaud's syndrome, striae distensae, peritoneal adhesions, vasospasm, rhinorrhea, muscle contractures, laryngeal dystonia, writer's cramp, and carpal tunnel syndrome.

22. A cosmetic composition for preventing or improving of neuromuscular related disease, comprising the pharmaceutical liquid composition of Claim 1 as an active ingredient.

23. A method for preventing or treating of neuromuscular related disease, comprising the step of administering the pharmaceutical liquid composition of Claim 1 to a subject in need thereof.

24. Use of the pharmaceutical liquid composition of Claim 1 for preventing or treating of neuromuscular related disease.

25. Use of the pharmaceutical liquid composition of Claim 1 for manufacturing a therapeutic agent for neuromuscular related disease.
